(19) **Europäisches Patentamt**
**European Patent Office**
**Office européen des brevets**

(11) **EP 3 378 381 A1**

(12) **EUROPEAN PATENT APPLICATION**

(43) Date of publication:
26.09.2018 Bulletin 2018/39

(51) Int Cl.:
*A61B 5/00* (2006.01)          *A61B 5/026* (2006.01)
*A61B 5/145* (2006.01)          *A61B 18/00* (2006.01)

(21) Application number: 17162723.5

(22) Date of filing: 24.03.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventor: **MUELLER, Manfred**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **INTRAVASCULAR BLOOD FLOW DETERMINATION BASED ON VORTEX SHEDDING**

(57)     A signal processing unit (208) for determining a value of a blood flow quantity characterizing blood flow inside a blood vessel comprises a vibration sensor signal input, which is configured to receive a vibration sensor signal from an intravascular blood flow sensor, the vibration sensor signal comprising a vibration sensor signal component caused by vortex-generated blood flow oscillations of intravascular blood flow, and a blood flow determination unit which is configured to determine the vibration sensor signal component using the vibration sensor signal, to determine an oscillation frequency of the vortex-generated blood flow oscillations using the vibration sensor signal component and to determine and provide the value of the blood flow quantity using the determined oscillation frequency.

FIG. 1A

FIG. 1B

**Description**

FIELD OF THE INVENTION

The present invention is in the field of hemodynamic sensing, especially in the sensing of blood flow related parameters. In particular, it relates to a signal processing unit, to an intravascular blood flow sensor for measuring blood flow inside a blood vessel, to an intravascular blood flow sensor system, to method for controlling operation of an intravascular blood flow sensor system, to a method for operating a signal processing unit for determining a value of a blood flow quantity characterizing blood flow inside a blood vessel, and to a computer program.

BACKGROUND OF THE INVENTION

US 2014/0276137 A1 describes systems and methods for determining coronary flow reserve (CFR) using a flow reserve index obtained at rest and during hyperemia. A method described therein includes obtaining a resting value for a flow reserve index from a patient, obtaining a hyperemic value for the flow reserve index from the patient, computing the coronary flow reserve based on the resting value and the hyperemic value, and providing the coronary flow reserve to a user.

SUMMARY OF THE INVENTION

It would be desirable to provide an alternative way for determination of a blood flow quantity.
According to a first aspect of the present invention, a signal processing unit for determining a value of a blood flow quantity characterizing blood flow inside a blood vessel is provided. The signal processing unit comprises:

- a vibration sensor signal input, which is configured to receive a vibration sensor signal from an intravascular blood flow sensor, the vibration sensor signal comprising a vibration sensor signal component caused by vortex-generated blood flow oscillations of intravascular blood flow; and
- a blood flow determination unit which is configured,
- using the vibration sensor signal, to determine the vibration sensor signal component;
- using the vibration sensor signal component, to determine an oscillation frequency of the vortex-generated blood flow oscillations; and,
- using the determined oscillation frequency of the vortex-generated blood flow oscillations, to determine and provide the value of the blood flow quantity.

The signal processing unit for determining a value of a blood flow quantity characterizing blood flow inside a blood vessel allows a particularly fast, easy, and reliable determination of a blood flow quantity.

The signal processing unit is based on the recognition that blood flow quantities can be determined using a vibration sensor signal, and in particular a vibration sensor signal component that is caused by vortex-generated blood flow oscillations of intravascular blood flow. With a suitable intravascular device that influences the flow pattern and velocity of blood flow, turbulences in the blood flow are caused which are indicated by the vibration sensor signal component. While known principles of flow sensor operation try to minimize this effect by using streamlined shapes and minimizing the size of the intravascular device, the inventor found out that it is in fact possible to use vibration sensor signals indicative of such disturbances in the blood flow caused by the intravascular device or (and) the vessel structure to determine blood flow quantities such as a blood flow velocity with the aid of the signal processing unit of the first aspect of the present invention.
In particular, the signal processing unit receives, at a vibration sensor signal input, the vibration sensor signal from an intravascular vibration sensor. The vibration sensor is not part of the signal processing unit. It may form a part of an external blood flow sensor that provides the vibration sensor signal to the signal processing unit. In the signal processing unit of the first aspect, the blood flow determination unit is connected to the vibration sensor signal input and determines, using the vibration sensor signal, the vibration sensor signal component that is caused by vortex-generated blood flow oscillations of intravascular blood flow. Blood flow oscillations generated by the vortices that are suitably generated inside the vessel cause a vibration sensor signal component of the vibration sensor signal. Other signal components include those caused for example by blood flow alterations due to heartbeat, or by relative movement of the intravascular blood flow sensor with respect to the living being in whose vessel the sensor is located.
The blood flow determination unit also determines, using the vibration sensor signal component, an oscillation frequency of the vortex-generated blood flow oscillations, and further determines and provides the value of the blood flow quantity, using the determined oscillation frequency.
Thus, a new source of information on blood flow quantities is opened up by the by signal processing unit of the present invention. In the following, embodiments of the signal processing unit will be described.
Several blood flow quantities are known, which can be determined using the signal processing unit. For instance, a volume flow quantity, given for instance in units of in ml/s, or a flow velocity quantity, expressed in units of m/s, or a coronary flow reserve (CFR) can be determined.
In some embodiments of the signal processing unit, the blood flow determination unit comprises a signal transformation unit, which is configured to determine a frequency-domain representation of the vibration sensor signal received during a predetermined measuring time span and to determine the oscillation frequency of the vortex-generated blood flow oscillations using the frequency-domain representation. The frequency-domain representation can be determined and provided for instance by a signal transformation unit applying a Fourier transform, suitably a Fast Fourier Transform (FFT) of the received vibration sensor signal.

For typical coronary flows and geometry an oscillation frequency associated with the vortices of a few 100 Hz is to be expected. However, this will be superimposed with other frequencies caused by heart beat (around 1 Hz) or other disturbances. In preferred embodiments, therefore, the blood flow determination unit of the signal processing unit comprises a filter unit configured to filter out frequency components of the vibration sensor signal that are associated with a heartbeat frequency. In one example, after subjecting the vibration sensor signals to an FFT all frequency components of the vibration sensor signal smaller than 100 Hz are attenuated or fully eliminated from the vibration sensor signal by the filter unit. The remaining vibration sensor signal components in the desired frequency range can then be used to identify a frequency component associated with the vortex-generated oscillations. Suitably, the vibration sensor signal component having the strongest amplitude to the remaining filtered signal comprising frequency components above 100 Hz can be identified as that associated with vortex-generated oscillations. Thus, by providing a frequency filtering, it is made sure that the frequencies significant for determination of the blood flow quantity are identified and selected for further signal processing.

In order to provide an absolute value of a blood flow velocity as the value of the blood flow quantity, geometrical data such as a characteristic size of the blood vessel at a measurement position of the vibration sensor is provided in some embodiments. In such embodiments, the blood flow determination unit is preferably further configured to hold or receive the geometrical data indicative of the characteristic size of the blood vessel at the intravascular position of the vibration sensor. In general, for intravascular applications, the can be characteristic size is equivalent to a hydraulic diameter, which is a common quantity used in the characterization of flow in channels of non-circular cross section. For blood vessels of nearly circular diameter, the hydraulic diameter can be approximated by the diameter of the tube or channel.

In some embodiments, the blood flow determination unit is further configured to hold or receive geometrical data indicative of a characteristic size of the blood vessel at a current intravascular position of the vibration sensor during measurement. The geometrical data is in some embodiments held or stored in a storage unit, whereas in other embodiments, the geometrical data is received by an input unit. The input unit may for instance be a user interface allowing manual input of the geometrical data. In yet other embodiments, the geometrical data is provided by an external image-processing device configured to determine the characteristic size from image data taken of the blood vessel in-situ, i.e., at the current intravascular position of an intravascular blood flow sensor comprising the vibration sensor.

In some of these embodiments, the blood flow determination unit is configured to determine and provide, using the determined oscillation frequency of the vortex-generated blood flow oscillation and the geometrical data, the value of the blood flow quantity as a flow velocity according to:

$$v = \frac{f \cdot d}{S} \ ,$$

wherein

> $v$ is the flow velocity;
> $f$ is the determined oscillation frequency of the vortex-generated blood flow oscillation;
> $d$ is the characteristic size of the blood vessel; and
> $S$ is a constant representing the Strouhal number applicable to blood flow in the given blood vessel.

The Strouhal number is a dimensionless number that describes oscillating flow mechanisms. For a range of Reynolds number covering the interval applicable for blood, the Strouhal number is suitably approximated by a constant value, suitably a value of 0.2. At this value of the Strouhal number, oscillations in fluid flow are characterized by a buildup and subsequent rapid shedding of vortices in the presence of a bluff body inside the blood vessel, such as a suitably shaped blood flow sensor, which will be described further below.

Other embodiments of the signal processing unit of the first aspect make use of the recognition of the present inventor that relative changes in blood flow over time allow determining values of a flow velocity ratio despite an unknown geometry and size of the blood vessel, in which the blood flow is to be measured. In particular, the inventors has recognized that use can be made of the fact that the parameters required for determining the value of the blood flow quantity the characteristic size of the blood vessel, are sufficiently stable over time, even if not known in absolute values, as long as the intravascular device is not moved during the measurement. This recognition opens up embodiments of blood flow measurements, in which the vibration sensor provides respective vibration sensor signals comprising the vibration sensor signal component caused by vortex-generated blood flow oscillations of intravascular blood flow measured at two different measuring times. In such embodiments, the signal processing unit, which receives the vibration sensor signals, is configured to determine respective oscillation frequencies of the vortex-generated blood flow oscillations at at least two different measuring times and to determine and provide as an output a frequency ratio of the determined oscillation frequencies at the two measuring times as the value of the blood flow quantity. The determination of a frequency ratio ($r$) of the determined oscillation frequencies at the two measuring times, allows direct conclusions on blood flow quantities such as a flow velocity ratio or, in particular embodiments, a coronary flow reserve (CFR) with particular ease and reliability. In particular, based on the applicability of the assumptions explained above, the flow velocity ratio is identical to the ratio of the measured oscillation frequencies at two different times, as it is show in the following equation:

$$\frac{v_B}{v_A} = \frac{f_B \cdot S/d}{f_A \cdot S/d} = \frac{f_B}{f_A} = r \, ,$$

wherein

$v_A$ and $v_B$ are values of blood flow velocities of blood inside the vessel at two different times $A$ and $B$; and $f_A$ and $f_B$ are oscillation frequencies determined at the two different times.

Such blood flow quantities provide important information regarding the current physiological state of a blood vessel, and advantageously assist in particular in the identification and quantitative characterization of a stenosis or of the coronary microcirculation.

As mentioned, the signal processing unit of one embodiment is configured to determine a value of a coronary flow reserve (CFR) from the frequency ratio, using respective received vibration sensor signals at a first measuring time corresponding to a state of normal blood flow, in particular at a time of rest of the patient, and at a second measuring time corresponding to a state of hyperemia. The blood flow determination unit is configured to determine and output, in particular display a value of a coronary flow reserve from the frequency ratio the CFR value by determining the ratio between the oscillation frequencies determined for the two different states. However, this is only a special example. Any other flow velocity ratio maybe determined using corresponding measurements at any two different states, generally referred to as a state A and a state B.

In some of these embodiments, the blood flow determination unit is configured to determine the frequency ratio of the determined oscillation frequencies at the two measuring times as an average value from frequency ratios of the determined oscillation frequencies at respective two measuring times of a plurality of measurement iteration cycles. This can be done by measuring the vibration sensor signal over one heart cycle or multiple heart cycles or in a time resolved way. Generally, and especially where the CFR shall be determined and state A is a state of normal blood flow and state B a state of hyperemia, it is better for the patient to induce each state only once and perform a respective plurality of measurements for obtaining a suitable number of frequency samples in each of the two states A and B. If circumstances allow, the measurements may be determined by performing two or more iteration cycles, i.e., iteratively changing between the states A and B.

To provide a user with a possibility to control the individual measuring times, the signal processing unit of some embodiments additionally comprises a user interface. The user interface is preferably configured to allow a user triggering a measurement and providing a first vibration sensor signal associated with a first measuring time. The blood flow determination unit, in response to receiving the user input, is configured to receive a sequence of vibration sensor signals at different measuring times, and to determine and provide the frequency ratio of the determined oscillation frequencies for the given measuring times with respect to the oscillation frequency determined from the first vibration sensor signal. Typically, in operation of signal processing unit, a user such as medical staff selects the first measuring time and from then on the signal processing unit provides the current value of the blood flow quantity as relative values. In other embodiment, the user marks both measuring times corresponding to states A and B by suitable user input.

In some of these embodiments, the signal processing unit is further configured to provide relative changes in blood flow over time in comparison with a user-defined reference point in time. In such embodiments, the signal processing unit, in response to receiving the user input, is suitably configured to receive a sequence of vibration sensor signals, beginning at the user-defined first measuring time and then at subsequent measuring times after the first measuring time, which may be quasi-continuous or determined automatically by a preset sampling frequency. The signal processing unit determines and provide the respective frequency ratio of the determined oscillation frequencies for the different measuring times with respect to the oscillation frequency determined from the first vibration sensor signal. In other words, the user can select the first measuring time or first measuring time span for which the blood flow is to be considered 100%, and the signal processing unit will after that determine and provide current blood flow as relative values with reference to the blood flow at that selected first measuring time.

According to a second aspect of the present invention an intravascular flow sensor is provided. The intravascular flow sensor comprises:

- a guidewire or catheter for intravascular insertion having a bluff part that is shaped for generation of vortices propagating along a main direction of intravascular blood flow; and
- a vibration sensor arranged and configured to provide the vibration sensor signal indicative of the oscillation frequency of the vortex-generated blood flow oscillations.

The intravascular blood flow sensor of the second aspect of the invention is advantageously configured to provide the vibration sensor signal that comprises a vibration sensor signal component caused by vortex-generated blood flow oscillations of intravascular blood flow and is preferably used in combination with the signal processing unit of the first aspect of the invention as it will be discussed below with reference to a third aspect of the present invention.

The blood flow in the coronary arteries has a reported Reynolds number between 50 and 1000. The inventors make use of the per-se known fact that fluids having Reynolds numbers typically larger than 50 tend to exhibit vortex shedding when the fluid moves past a suitably shaped bluff part of the intravascular blood flow sensor, such as a bluff or a barrier on or in its body, as opposed to an intravascular blood flow sensor having a body of streamlined shape. Such a suitably shaped part of a catheter or guidewire is provided in the intravascular blood flow sensor. This bluff part may in different embodiments be a part of an intravascular guidewire or catheter, in particular micro-catheter.

Vortex shedding, as mentioned, describes a periodic formation of vortices, also known as Kármán vortices, behind the bluff part of the catheter or guidewire comprised by the intravascular blood flow sensor, wherein "behind the bluff part" refers to a view in a main direction of blood flow at the bluff part. The vortices propagate along a main direction given by the blood flow direction. At any given time, the vortices are distributed behind the bluff part showing a respective spatial distribution. Generally, vortex-generated blood flow oscillations of intravascular blood flow can be detected in a direction substantially perpendicular to a main direction of blood flow along the blood vessel.

In the following, embodiments of the second aspect of the present invention will be described. These different embodiments are based on different techniques for providing the vibration sensor signals.

The flow determination makes use of the fact that the frequency of vortex shedding is a measurable quantity that is related to the flow velocity by parameters known as the Strouhal number and the characteristic size of the blood vessel guiding the blood flow.

In one group of embodiments, the frequency of the vibrations behind the bluff part is determined using a vibration sensor that comprises a flagellum. The flagellum extends from the catheter or guidewire of the intravascular blood flow sensor in the main direction of intravascular blood flow and is elastically deformable in the direction perpendicular to the main direction of intravascular blood flow by the vortex-generated blood flow oscillations. The flagellum is floppy so as not to pose any risk of damage to vascular tissue. In some of these embodiments, the flagellum is made of an electro-active polymer material. A flagellum of this kind is configured to generate and provide the vibration sensor signal in the form of a time-varying electrical signal having an amplitude depending on a current deformation amount in the direction perpendicular to the main direction of intravascular blood flow. From the oscillating amplitude of this electrical signal as a function of time, a frequency of vortex-generated oscillation can be determined.

Alternatively, an optoelectronic solution can be employed to measure the oscillation frequency. An example of embodiments of this kind has an optical fiber segment forming the flagellum, configured to receive and guide light to and from a reflective fiber-segment tip. The intravascular blood flood sensor of these embodiments suitably further comprises a light source that is configured to provide light for coupling into the fiber segment, and a light sensor arranged to receive light reflected from the fiber-segment tip and modulated in intensity by the oscillating deformation of the fiber segment. The light sensor is configured to provide the vibration sensor signal in the form of a light-sensor signal indicative of a time-varying light intensity received back from the reflective fiber-segment tip.

Thus, a modulation of the light intensity reflected from the fiber-segment tip provides an electrical vibration sensor signal that can be used for evaluation of the oscillation frequency of the vortex-generated blood flow oscillations.

In other embodiments, the bluff part forms a tip section of the guidewire or catheter for intravascular insertion and is itself elastically deformable in the direction perpendicular to the main direction of intravascular blood flow by the vortex-generated blood flow oscillations. Here, the vibration sensor is suitably arranged in the tip section of the guidewire and thus measures the vibration of the tip section of the guidewire or the catheter, i.e., its oscillation frequency in response to vortex-generated blood flow oscillations in a direction perpendicular to the main direction of intravascular blood flow.

In yet other embodiments, the vibration sensor comprises one or more pressure sensors arranged on a surface of the catheter or guidewire. The pressure sensor is arranged on the catheter or guidewire at a position behind the bluff part in the direction of blood flow, in order to sense pressure variations exerted by the blood flow and caused by the presence of the bluff part on the guidewire or catheter. The pressure sensor is configured to measure a time-varying pressure exerted in the direction perpendicular to the main direction of intravascular blood flow by the vortex-generated blood flow oscillations and to generate and provide the vibration sensor signal in the form of a time-varying electrical signal depending on the measured pressure. Thus, the pressure sensor of this embodiment is preferably arranged on a circumferential surface section of the intravascular blood flow sensor, and not on a front surface section at the tip of the guidewire or catheter. Some embodiments using pressure sensing are advantageously configured to additionally determine a value of a fractional flow reserve (FFR). This determination is based on a low frequency band of the time-varying electrical signal depending on the measured pressure and provided by the pressure sensor. Vortex-induced frequency components within the vibration sensor signal typically are at frequencies of a few 100 Hz, and are overlaid with low frequency signal components associated with a heartbeat. The latter components have a frequency of around 1 Hz. FFR can thus be determined from the low frequency pressure signal that depicts the pressure changes over the heart cycle while the blood flow ratios can be determined from the oscillation frequencies at a higher frequency band obtained at the two measuring times.

For creating vortex-generated blood flow oscillations, the bluff part that is shaped for generation of vortices propagating along a main direction of intravascular blood flow preferably comprises a barrier section that protrudes from the guidewire or catheter in the direction perpendicular to the main direction of intravascular blood flow. In particular embodiments, the barrier section forms a bluff body section, such as a ball-shaped body section. Generally, any non-streamlined shape can be used to encourage the formation of the vortices.

According to a third aspect of the present invention, an intravascular blood flow sensor system is provided. The intravascular blood flow sensor system comprises an intravascular blood flow sensor according to the second aspect of the invention or any of its embodiments and a signal processing unit according to the first aspect of the present invention or any of its embodiments.

In general, the intravascular blood sensor system of the third aspect includes an intravascular blood flow sensor system, which includes an intravascular blood flow sensor that comprises:

- a guidewire or catheter for intravascular insertion having a bluff part that is shaped for generation of vortices propagating along a main direction of intravascular blood flow; and
- a vibration sensor arranged and configured to provide the vibration sensor signal indicative of the oscillation frequency of the vortex-generated blood flow oscillations;
- the blood flow sensor system further including a signal processing unit ac-cording to the first aspect of the invention or one of its embodiments.

In the following, embodiments of the intravascular blood sensor system of third aspect of the present invention will be described. These embodiments share the respective advantages of the corresponding embodiments of the first and the second aspect of the invention.

In some embodiments of the intravascular blood flow sensor system, the vibration sensor is arranged in a tip section of the guidewire or catheter.

In some of these embodiments the tip section is elastically deformable in the direction perpendicular to the main direction of intravascular blood flow by the vortex-generated blood flow oscillations.

In other embodiments of the intravascular blood sensor system the vibration sensor comprises a flagellum that extends from the catheter or guidewire in the main direction of intravascular blood flow and is elastically deformable in the direction perpendicular to the main direction of intravascular blood flow by the vortex-generated blood flow oscillations.

In a subset of these embodiments the flagellum is made of an electro-active polymer material and configured to generate and provide the vibration sensor signal in the form of a time-varying electrical signal having an amplitude depending on a deformation amount in the direction perpendicular to the main direction of intravascular blood flow.

In another subset of these embodiments the flagellum is an optical fiber segment configured to receive and guide light to and from a reflective fiber-segment tip, and the intravascular blood flow sensor system further comprises a light source configured to provide light for coupling into the fiber segment and a light sensor arranged to receive light reflected from the fiber-segment tip and modulated in intensity by oscillating deformation of the fiber segment, the light sensor being configured to provide the vibration sensor signal in the form of a light-sensor signal indicative of a time-varying reflected light intensity.

In yet other embodiments of the third aspect, the vibration sensor comprises one or more pressure sensors arranged on a surface of the catheter or guidewire, the pressure sensor being arranged and configured to measure a time-varying pressure exerted in the direction perpendicular to the main direction of intravascular blood flow by the vortex-generated blood flow oscillations and to generate and provide the vibration sensor signal in the form of a time-varying electrical signal depending on the measured pressure.

In other embodiments, the bluff part comprises a barrier section that protrudes from the catheter or guidewire in the direction perpendicular to the main direction of intravascular blood flow for generation of vortices propagating along the main direction of intravascular blood flow.

In other embodiments, the intravascular blood flow sensor system further comprises a signal communication unit configured to receive the vibration sensor signals and to transmit the vibration sensor signals via a wireless carrier signal to the signal processing unit. In these embodiments, the signal processing unit is further configured to extract the vibration sensor signals from the carrier signal.

The intravascular blood flow sensor can be implemented as an add-on device that can be mechanically and electrically mounted to a guidewire or catheter shaft. In preferred embodiments, however, at least the bluff part that is shaped for generation of vortices propagating along a main direction of intravascular blood flow intravascular flow sensor, and the vibration sensor arranged and configured to provide a vibration sensor signal indicative of the oscillation frequency of vortex-generated blood flow oscillations, form an integral part of a guidewire or catheter for intravascular insertion.

The signal processing unit is preferably located outside a guidewire or catheter comprised by the intravascular blood flow sensor. When implemented as such a unit of an external control and/or evaluation device that is not for intravascular insertion, the signal processing unit is suitably provided in the form of a programmed processor unit and configured to be in communicative connection with the vibration sensor during intravascular operation for receiving the vibration sensor signals via a wired or wireless communication channel, implementations of which are per se known in the art. Thus the intravascular part of the intravascular blood flow sensor also comprises suitable communication unit for wired or wireless communication of the vibration sensor signals.

Thus, some embodiments of the intravascular blood flow sensor additionally comprise a signal communication unit that is configured to receive the vibration sensor signals and to transmit the vibration sensor signals as a wired or wireless, i.e., electrical or electromagnetic signal to the signal processing unit, in particular using a suitable carrier signal where useful. In these embodiments, the signal processing unit is further configured to extract the vibration sensor signals from the carrier signal. Different embodiments make use of different wireless communication techniques, such as those based on any of the IEEE 802.11 standards (WiFi, WLAN), ZigBee, Bluetooth, wireless communication in an infrared frequency band, etc. The choice of the wireless communication technique inter alia depends on whether the signal communication unit is for intravascular use or for use outside the living being. In the former case, wireless radio communication techniques such are preferred. In the latter case, other wireless communication techniques such as those based on infrared data transmission may also be used.

In other embodiments, the intravascular blood flow sensor is arranged on or embedded in an intravascular ultrasound device for intravascular ultrasound imaging.

According to a fourth aspect of the present invention, a method for operating a signal processing unit for determining a value of a blood flow quantity characterizing blood flow inside a blood vessel is presented. The method comprises:

- receiving a vibration sensor signal from an intravascular blood flow sensor, the vibration sensor signal comprising a vibration sensor signal component caused by vortex-generated blood flow oscillations of intravascular blood flow;
- determining, using the vibration sensor signal, the vibration sensor signal component;
- determining, using the vibration sensor signal component, the oscillation frequency of the vortex-generated blood flow oscillations; and
- determining, using the determined oscillation frequency of the vortex-generated blood flow oscillations, and providing the value of the blood flow quantity.

According to a fifth aspect of the present invention, a method for controlling operation of an intravascular blood flow sensor system is provided. The method comprises:

- providing an intravascular blood flow sensor for measuring blood flow inside a blood vessel, the intravascular blood flow sensor comprising a guidewire or catheter for intravascular insertion having a bluff part that is shaped for generation of vortices propagating along a main direction of intravascular blood flow and a vibration sensor arranged and configured to provide a vibration sensor signal indicative of an oscillation frequency of vortex-generated blood flow oscillations;
- measuring, at two different measuring times, a vibration sensor signal from the vibration sensor;
- determining respective oscillation frequencies of the vortex-generated blood flow oscillations at the two different measuring times;
- determining a frequency ratio of the determined oscillation frequencies at the two measuring times.

The method of the fifth aspect shares the advantages signal processing unit of the first aspect and of the intravascular blood flow sensor of the second aspect of the invention.

A sixth aspect of the present invention is formed by a computer program comprising executable code for performing a method of the fourth aspect of the invention when executed by a programmable processor of a computer.

A seventh aspect of the present invention is formed by a computer program comprising executable code for performing a method of the fifth aspect of the invention when executed by a programmable processor of a computer.

It shall be understood that signal processing unit of claim 1, the intravascular blood flow sensor system of claim 6 and the method for operating a signal processing unit for determining a value of a blood flow quantity characterizing blood flow inside a blood vessel of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1a shows a schematic representation of a flow of a medium around stream-line shaped object;

Fig. 1b shows a schematic representation of the same flow of the medium around a bluff or barrier generating vortices in the flow;

Fig. 2 illustrates an embodiment of an intravascular blood flow sensor system comprising a signal processing unit and an intravascular blood flow sensor;

Fig. 3 shows another embodiment of an intravascular blood flow sensor system;

Fig. 4 shows another embodiment of an intravascular blood flow sensor system;

Fig. 5 shows another embodiment of an intravascular blood flow sensor system;

Fig. 6 is a flow diagram of a method for controlling operation of an intravascular blood flow sensor; and

Fig. 7 is a flow diagram of a method for operating a signal processing unit for determining a value of a blood flow quantity characterizing blood flow inside a blood vessel.

DETAILED DESCRIPTION OF EMBODIMENTS

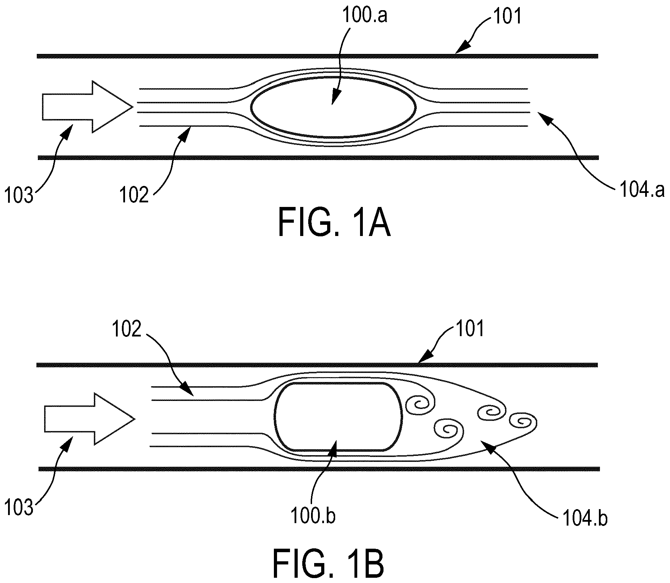

Fig. 1a and Fig. 1b show schematic illustrations of blood flow around a stream-line shaped object 100.a and around a bluff body 100.b in a blood vessel 101 at a fixed time. An incoming blood flow 102 in a main direction of blood flow that is indicated by the arrows 103 is the same in both figures and generally illustrated by straight flow lines upstream of the bluff body. In Fig.1a, a stream-lined shape of the object 100.a does not generate vortices in the blood flow behind the object 104.a. In the case of Fig. 1b, the bluff body 100.b generates vortex shedding in the blood flow behind it.

Generally, vortex shedding is known per se as an oscillating flow that occurs under suitable circumstances when a fluid flows past a bluff body. The parameters relevant for vortex shedding to occur comprise a viscosity of the fluid, a flow velocity, as well as a size and shape of the object. The former can be characterized, for example, by a Reynolds number. The vortex shedding induced by the presence of the bluff body 100.b in the blood flow 102 generates a so-called Kármán vortex street 104.b downstream of the bluff body 100.b. Existing vortices propagate to positions further away from the bluff body along the main direction of blood flow indicated by the arrows 102, while new vortices are generated close to the body 100.b. Vortices are generated at alternating sides of the body and are associated with oscillations in the blood flow in a direction perpendicular to the main flow direction. At a given time, the vortices generated are distributed as exemplarily shown in Fig.1b.

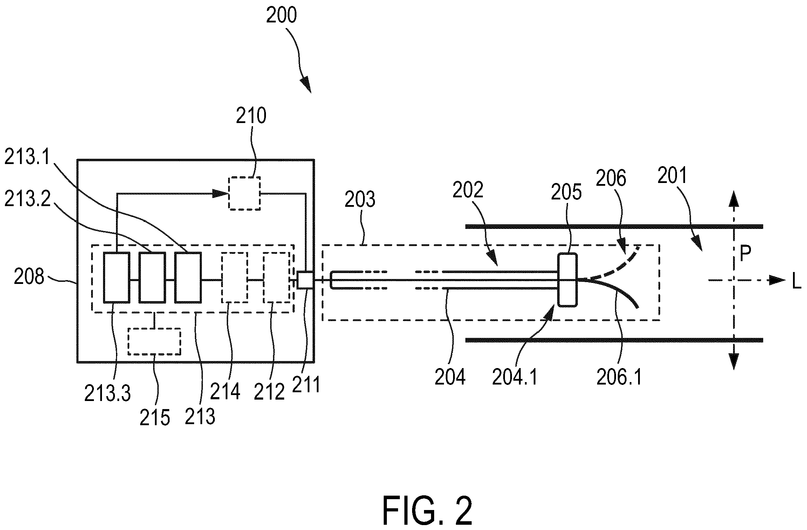

Fig. 2 is a schematic illustration of an embodiment of an intravascular blood flow sensor system 200 for measuring blood flow inside a blood vessel 201. The intravascular blood flow sensor 200 comprises an intravascular blood flow sensor 203 that includes an intravascular guidewire 202 that has a guidewire body 204 with an atraumatic tip section 204.1 comprising a bluff part 205 that is suitably shaped for generation of vortices propagating along a main direction L of intravascular blood flow. It is noted that the bluff part 205 of the intravascular blood flow sensor need not necessarily be different in shape from other parts of the guide wire body 204 for enabling the formation of vortices. However, to facilitate reliable generation of vortices even at low blood flow velocities, it is advantageous to add features that shape the body of a typical guide wire or catheter in a less stream-lined way, such as for example providing a guidewire or a catheter comprising a bluff part.

The guidewire body 204 may have a rotational symmetry along its longitudinal direction, which in Fig. 2 corresponds to the direction L. However, in other embodiments (not shown), the generation of vortices is alternatively or additionally made possible or enhanced by providing a shape of the microcatheter or guidewire that exhibits a break of a rotational symmetry in at least part of the tip.

The tip section 204.1 includes a vibration sensor 206. The vibration sensor 206 comprises a flagellum 206.1 extending from a front surface of the tip section 204.1 in the main direction L of the intravascular blood flow. The flagellum 206.1 is elastically deformable in a direction P perpendicular to L which in the present example are the two mutually opposite directions P. An oscillating bending motion of the flagellum 206.1 in the direction P is driven by the vortex-generated oscillating motion of blood, as explained with reference to Fig. 1b. At a given time, the propagating vortices thus show a respective distribution that alternates vortices at different downstream positions of the tip section 204.1 in the longitudinal direction L (as exemplarily shown in Fig. 1b). Vortex-generated oscillations may occur in any direction that is perpendicular to the longitudinal direction L.

In a real 3D vessels, more complex vortex configurations are possible that cannot be faithfully represented in a 2D illustration such as Figs. 1a and 1b. The vibration sensor is thus configured to provide a vibration sensor signal indicative of the blood flow oscillations, but not necessarily of the propagation direction of the vortices.

The flagellum 206.1 is shown in Fig. 2 in two different phases of an oscillating bending motion corresponding to two different bending positions of the flagellum 206.1. A first phase of the oscillating motion is represented by a solid line, and a second phase is represented by a dotted line.

The flagellum 206.1 comprised by the vibration sensor 206 can be made of an electro-active polymer material and configured to generate and provide the vibration sensor signal in the form of a time-varying electrical signal having an amplitude depending on a deformation amount in the direction perpendicular to the main direction of intravascular blood flow.

The intravascular blood flow sensor 200 also comprises a signal processing unit 208 for determining a value of a blood flow quantity characterizing blood flow inside a blood vessel. The signal processing unit comprises a vibration sensor signal input 211 receives the vibration sensor signals from the vibration sensor of the intravascular blood flow sensor. The vibration sensor signal comprises a vibration sensor signal component caused by vortex-generated blood flow oscillations of intravascular blood flow. In general, the vibration sensor signal component caused by the vortex-generated blood flow oscillations is a component in a direction perpendicular to the main direction of blood flow. The signal processing unit 208 further comprises a blood flow determination unit 213 with is configured to determine the vibration sensor signal component using the vibration sensor signal, to determine the oscillation frequency of the vortex-generated blood flow oscillations using the vibration sensor signal component, and to determine and provide the value of the blood flow quantity using the determined oscillation frequency. In this exemplary signal processing unit 208 these three distinct tasks are performed by three respective units 213.1, 213.2 and 213.3. In other signal processing units, the three described tasks are performed by a processor.

Some signal processing units include a blood flow determination unit that additionally comprises a signal transformation unit (212), which is configured to determine a frequency-domain representation of the vibration sensor signal received during a predetermined measuring time span and to determine the oscillation frequency of the vortex-generated blood flow oscillations using the frequency-domain representation. To this end, the signal transformation unit 212 receives the vibration sensor signals from the vibration sensor signal input over a predetermined measuring time span associated with a given measuring time. The signal transformation unit 212 determines the oscillation frequency for the given measuring time using a frequency-domain representation of the vibration sensor signal during the respective measuring time span. Suitably, the signal transformation unit 212 is a Fast Fourier Transform unit that determines the Fourier Transform of the vibration sensor signal. From the transformed vibration sensor signal, an oscillation frequency can be determined in a simple manner as a frequency of a Fourier component having a maximum amplitude in an expected oscillation frequency range above 100 Hz, typically in the range of a few hundred Hz.

To make detection of the oscillation frequency easier, some signal processing units of the present embodiment further comprises a filter unit 214 that is configured to filter out frequency components from the vibration sensor signal that are associated with heart beat frequency. The heart beat frequency range is typically below 100 Hz.

Some signal processing unit further determines a frequency ratio of the determined oscillation frequencies at two measuring times. This way, blood flow quantities can be determined. Such blood flow quantities provide important information regarding the current physiological state of a blood vessel, and assist in the identification and quantitative characterization of a stenosis. For calculation and output of the CFR value, the measurements are made in a state of hyperemia and in a state of normal blood flow (e.g., at rest). The coronary flow reserve (CFR) is then determined and provided by the signal-processing unit with particular ease and reliability as the frequency ratio of respective vibration sensor signals at the measuring time corresponding to the state of hyperemia and at the measuring time corresponding to the state of normal blood flow.

In some signal processing units, a user interface 210 is provided for user input of control signals, such as for triggering the oscillation measurements by controlling the operation of the vibration sensor signal input, and for output of the value of the blood flow quantity determined. The user interface is in some embodiments used to provide geometrical data indicative of a characteristic size of the blood vessel at a current intravascular position of the intravascular blood flow sensor, which is required to provide a value of a flow velocity according to:

$$v = \frac{f \cdot d}{S},$$

wherein

v is the flow velocity;
f is the determined oscillation frequency of the vortex-generated blood flow oscillation; d is the characteristic size of the blood vessel; and
S is a constant representing the Strouhal number applicable to blood flow in the given blood vessel.

In other signal processing units, the geometrical data is locally stored in a storage unit 215 which is accessed by the blood flow determination unit 213 for determining the value of the flow velocity v.

In other embodiments, the signal processing unit receives the geometrical data from an external imaging device or an external image processing device that is configured to image the blood vessel at a current intravascular position of the intravascular blood flow sensor and to determine and provide the geometrical data at that position.

As another mode of operation, which is available to a user as an alternative to the CFR determination, the signal processing unit 208 determines and provides relative changes in blood flow over time from a sequence of measurements, as compared to a first measurement of the sequence that can be triggered by user input.

In a variant of the intravascular blood flow sensor of Fig. 2, the flagellum comprised by the vibration sensor 206 is an optical fiber segment configured to receive and guide light to and from a reflective fiber-segment tip. These particular intravascular flow sensors also comprise a light source that is configured to provide light for coupling into the fiber segment and a light sensor arranged to receive light reflected from the fiber-segment tip and modulated in intensity by oscillating deformation of the fiber segment. The light sensor is configured to provide the vibration sensor signal in the form of an electronic light-sensor signal indicative of a time-varying reflected light intensity.

A further variant of the intravascular blood flow sensor of Fig. 2, which is not shown, comprises, instead of the guidewire 202, a microcatheter provided with the flagellum-type vibration sensor 206 in its tip section. The above description is otherwise equally applicable to that variant.

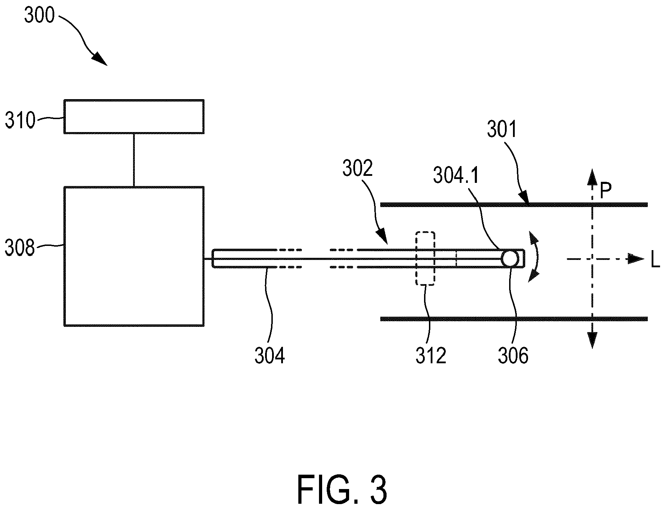

Fig. 3 illustrates another exemplary embodiment of an intravascular blood flow sensor 300 for measuring blood flow inside a blood vessel 301. The blood flow sensor 300 comprises a micro-catheter for intravascular insertion. A catheter body 304 of the micro-catheter may have a rotational symmetry along its longitudinal direction, which in Fig. 3 corresponds to the direction L. A tip section 304.1 of the catheter body 304 forms a bluff body part and is suitably shaped for generation of vortices propagating along a main direction L of intravascular blood flow. The tip section 304.1 is elastically deformable by vortex-generated blood flow oscillations in the directions P perpendicular to the main direction L of intravascular blood flow. A vibration sensor 306 is arranged in the tip section 304.1. The vibration sensor 306 is a motion sensor, suitably an acceleration sensor. As such, it provides an electrical sensor signal indicative of an oscillatory bending motion of the tip section 304.1 driven by the vortex-generated oscillations of blood flow in the vessel 301 at the location of the tip section 304.1. This sensor signal thus forms a suitable vibration sensor signal that is indicative of the oscillation frequency of the vortex-generated blood flow oscillations that propagate in the direction L.

The vibration sensor signals are provided by the vibration sensor and received by a signal processing unit 308, which is arranged outside the body of the patient. Details of signal communication and signal processing have been described in the context of the embodiment of Fig. 2 and are applicable here as well. A user may interact with the intravascular flow sensor 300 via a user interface 310, as also described above in more detail with reference to Fig. 2.

A variant of the intravascular blood flow sensor of Fig. 3, which is not shown, comprises, instead of the microcatheter 302, a guidewire provided with the vibration sensor 306 in its tip section. The above description is otherwise equally applicable to that variant.

A variant of the intravascular blood flow sensor of Fig. 3 comprises an additional bluff part 312 in the microcatheter body 304 of the microcathether 302. The bluff part 312 is arranged at a short distance from the tip section 304.1 in direction of the proximal end of the microcatheter 302. The presence of the bluff part 312 further enhances vortex shedding that induces a vibration of the tip section 304.1, where the vibration sensor 306 is arranged.

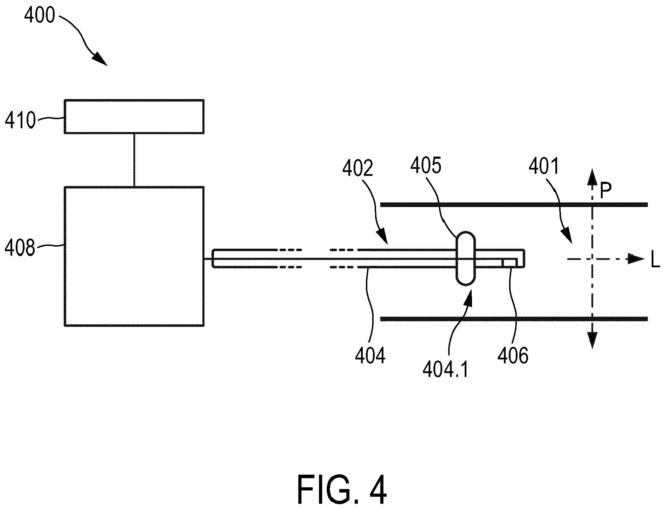

Fig. 4 illustrates another embodiment of an intravascular blood flow sensor 400 for measuring blood flow inside a blood vessel 401.

The intravascular blood flow sensor 400 comprises a microcatheter 402 with a catheter body 404 for intravascular insertion. A tip section 404.1 of the catheter body comprises a barrier section 405 that protrudes from the catheter body in a direction P perpendicular to the main direction of intravascular blood flow, in order to enhance the generation of vortices propagating along the main direction L of intravascular blood flow. Such a barrier 405 may also be present in the tip section in variants of the embodiments of Figs. 1 to 3. The shape of the barrier section 405 is only schematically indicated in Fig. 4. Any shape that is suitable to favor generation of vortices over laminar blood flow along the tip section 404.1 of the catheter body 404 can be used.

A vibration sensor is provided in the form of a pressure sensor 406 located on a surface of the tip section 404.1 of the catheter body 404. The pressure sensor 406 is arranged and configured to measure pressure exerted in the direction P perpendicular to the main direction L of the turbulent intravascular blood flow, and thus particularly detects the vortex-generated blood flow oscillations as corresponding pressure oscillations. The pressure sensor 406 generates a vibration sensor signal in the form of a time-varying electrical signal depending on the pressure currently sensed. The pressure sensor 406 provides the vibration sensor signal to a signal processing unit 408, using one of the signal communication techniques explained in the context of the embodiment of Fig. 2. A user may interact with the intravascular blood flow sensor 400 via a user interface 410, as also explained hereinabove.

Intravascular blood flood devices comprising one or more pressure sensors such as the pressure sensor 406 can additionally determine a value of a fractional flow reserve (FFR). Fractional flow reserve is the ratio of blood pressure after i.e., distal to a stenosis and the blood pressure before the stenosis. This determination is based on evaluating a low frequency band of the measured time-varying electrical signal. As mentioned, Vortex-induced frequencies within the vibration signal typically are at frequencies in the range of a few 100 Hz and are overlaid with low-frequency signal components associated with the heartbeat. The latter components have a frequency clearly below 100 Hz, typically around 1 Hz. FFR can thus be determined from the low frequency pressure signal that depicts the pressure changes over the heart cycle while CFR can be determined from the high frequency vortex-induced component.

A variant of the intravascular blood flow sensor of Fig. 4, which is not shown, comprises two pressure sensors on opposite sides of the of guide wire body 404. Then they can derive a flow sensing frequency signal by determining the differences between two pressure signals determined by the respective pressure sensors. The intravascular blood flow sensor is then configured to compute a blood pressure signal (for FFR) by averaging over the two signals determined by each of the two pressure sensors.

A variant of the intravascular blood flow sensor of Fig. 4, which is not shown, comprises, instead of the microcatheter 402, a guidewire provided with the pressure sensor 406 in its tip section. The above description is otherwise equally applicable to that variant.

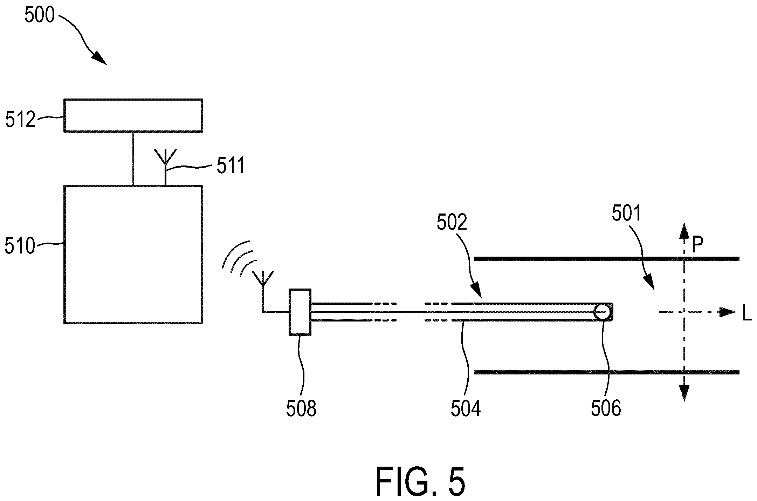

Fig. 5 shows a further embodiment of an intravascular blood flow sensor 500 in an inserted state inside a blood vessel 501. The blood flow sensor 500 comprises a guidewire 502 with a guidewire body 504. The blood flow sensor 500 also comprises a vibration sensor 506 implemented as any of the different kinds of vibration sensors discussed with reference to the embodiments of Figs 2-4.

The blood flow sensor 500 further comprises a signal communication unit 508 that is configured to receive the vibration sensor signals from the vibration sensor 506 and to perform wireless transmission of the vibration sensor signals to the signal processing unit 510 using a carrier signal. The signal processing unit 510 has a corresponding signal communication unit, of which only an antenna 511 is shown, that is configured to receive the carrier signal and to extract the vibration sensor signals from the carrier signal. The signal processing unit 510 then determines respective oscillation frequencies of vortex-generated blood flow oscillations at at least two different measuring times and determines and provides as an output a frequency ratio of the determined oscillation frequencies at the two measuring times. A user may interact with the blood flow sensor 500 via a user interface 512, as explained above. The user input may also be provided using wireless communication.

As shown in Fig. 5, the signal communication unit 508 is to be located outside the living being under examination. However, in a variant (not shown), the signal communication unit 508 is integrated into the guidewire body 504 and thus inserted in the blood vessel during operation. In these cases the transmission of the vibration sensor signals is suitably performed using radio communication protocols such as for example any of the IEEE 801.11 standards for wireless communication, a Bluetooth-based wireless communication protocol, or any other radio-based wireless communication protocol.

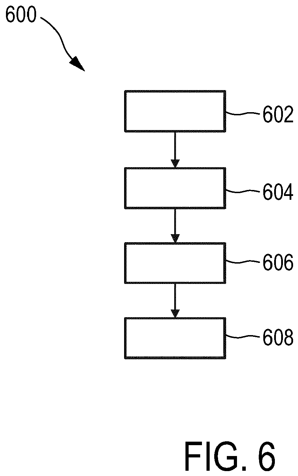

Fig. 6 shows a flow diagram of a method 600 for controlling operation of an intravascular blood flow sensor. The method comprises a step 602 in which an intravascular blood flow sensor for measuring blood flow inside a blood vessel is provided. The intravascular blood flow sensor comprises a guidewire or catheter for intravascular insertion having a bluff part that is shaped for generation of vortices propagating along a main direction of intravascular blood flow and a vibration sensor arranged and configured to provide a vibration sensor signal indicative of an oscillation frequency of vortex-generated blood flow oscillations in a direction perpendicular to the main direction of intravascular blood flow.

In a step 604, a vibration sensor signal is measured at two different measuring times using the vibration sensor. In a step 606, respective oscillation frequencies of the vortex-generated blood flow oscillations at the two different measuring times are determined. In a final step 608, a frequency ratio of the determined oscillation frequencies at the two measuring times is determined.

In summary, according to the present invention, an intravascular blood flow sensor for measuring blood flow inside a blood vessel comprises a guidewire or catheter for intravascular insertion having a bluff part that is shaped for generation of vortices distributed along a main direction of intravascular blood flow and It further comprises a vibration sensor arranged and configured to provide a vibration sensor signal indicative of an oscillation frequency of vortex-generated blood flow oscillations in a direction perpendicular to the main direction of intravascular blood flow. A signal processing unit receives the vibration sensor signals and determines respective oscillation frequencies of the vortex-generated blood flow oscillations at at least two different measuring times, and a frequency ratio of the determined oscillation frequencies at the two measuring times. By measuring vibration sensor signals in a state of hyperemia and in a normal state, a coronary flow reserve can be determined.

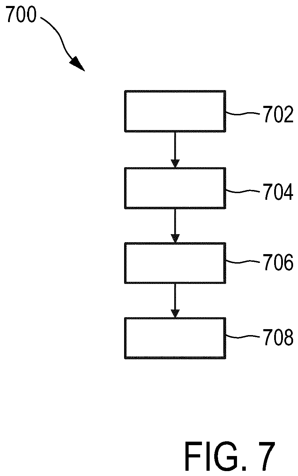

Fig. 7 shows a flow diagram describing a method 700 for operating a signal processing unit for determining a value of a blood flow quantity characterizing blood flow inside a blood vessel. The method comprises a step 702 in which a signal processing unit receives a vibration sensor signal from an intravascular blood flow sensor, the vibration sensor signal comprising a vibration sensor signal component caused by vortex-generated blood flow oscillations of intravascular blood flow. In a step 704, the signal processing unit determines the vibration sensor signal component using the vibration sensor signal. In a step 706, the signal processing unit determines the oscillation frequency of the vortex-generated blood flow oscillations using the vibration sensor signal component, and finally, in a step 708, the signal processing unit determines, using the oscillation frequency of the vortex-generated blood flow oscillations, and provides, the value of the blood flow quantity.

In summary, a signal processing unit for determining a value of a blood flow quantity characterizing blood flow inside a blood vessel comprises a vibration sensor signal input, which is configured to receive a vibration sensor signal from an intravascular blood flow sensor, the vibration sensor signal comprising a vibration sensor signal component caused by vortex-generated blood flow oscillations of intravascular blood flow, and a blood flow determination unit which is configured to determine the vibration sensor signal component using the vibration sensor signal, to determine the oscillation frequency of the vortex-generated blood flow oscillations using the vibration sensor signal component and to determine and provide the value of the blood flow quantity using the determined oscillation frequency.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1.  A signal processing unit (208) for determining a value of a blood flow quantity characterizing blood flow inside a blood vessel (201), the signal processing unit comprising:

    - a vibration sensor signal input (211), which is configured to receive a vibration sensor signal from an intravascular vibration sensor (203), the vibration sensor signal comprising a vibration sensor signal component caused by vortex-generated blood flow oscillations of intravascular blood flow; and
    - a blood flow determination unit (213) which is configured,

       - using the vibration sensor signal, to determine the vibration sensor signal component;
       - using the vibration sensor signal component, to determine an oscillation frequency of the vortex-generated blood flow oscillations; and,
       - using the determined oscillation frequency of the vortex-generated blood flow oscillations, to determine and provide the value of the blood flow quantity.

2.  The signal processing unit of claim 1, wherein the blood flow determination unit (213) comprises a signal transformation unit (212), which is configured to determine a frequency-domain representation of the vibration sensor signal received during a predetermined measuring time span and to determine the oscillation frequency of the vortex-generated blood flow oscillations using the frequency-domain representation.

3.  The signal processing unit of claim 2, wherein the blood flow determination unit (213) comprises a filter unit (214) configured to filter out frequency components of the vibration sensor signal that are associated with a heartbeat frequency.

4.  The signal processing unit of claim 1, wherein the blood flow determination unit is further configured to hold or receive geometrical data indicative of a characteristic size of the blood vessel at a current intravascular position of the intravascular vibration sensor.

5.  The signal processing unit of claim 1, wherein the blood flow determination unit is configured to determine respective oscillation frequencies of the vortex-generated blood flow oscillations at at least two different measuring times and to determine and provide as an output a frequency ratio of the determined oscillation frequencies at the two measuring times as the value of the blood flow quantity.

6.  An intravascular blood flow sensor system (200), which includes an intravascular blood flow sensor (203) that comprises:

    - a guidewire or catheter (202) for intravascular insertion having a bluff part (205) that is shaped for generation of vortices (104.b) propagating along a main direction (L) of intravascular blood flow; and
    - a vibration sensor (206) arranged and configured to provide the vibration sensor signal indicative of the oscillation frequency of the vortex-generated blood flow oscillations;
    - the blood flow sensor system further including a signal processing unit (208) according to claim 1.

7.  The intravascular blood flow sensor system of claim 6, wherein the vibration sensor (206) is arranged in a tip section (204.1) of the guidewire or catheter (204).

8.  The intravascular blood flow sensor system of claim 7, wherein the tip section (304.1) is elastically deformable in the direction perpendicular to the main direction of intravascular blood flow by the vortex-generated blood flow oscillations.

9.  The intravascular blood flow sensor system of claim 6, wherein the vibration sensor comprises a flagellum (206.1) that extends from the catheter or guidewire (204) in the main direction of intravascular blood flow and is elastically deformable in the direction perpendicular to the main direction of intravascular blood flow by the vortex-generated blood flow oscillations.

**10.** The intravascular blood flow sensor system of claim 9, wherein the flagellum is made of an electro-active polymer material and configured to generate and provide the vibration sensor signal in the form of a time-varying electrical signal having an amplitude depending on a deformation amount in the direction perpendicular to the main direction of intravascular blood flow.

**11.** The intravascular blood flow sensor system of claim 9, wherein the flagellum is an optical fiber segment configured to receive and guide light to and from a reflective fiber-segment tip; further comprising

- a light source configured to provide light for coupling into the fiber segment; and
- a light sensor arranged to receive light reflected from the fiber-segment tip and modulated in intensity by oscillating deformation of the fiber segment, the light sensor being configured to provide the vibration sensor signal in the form of a light-sensor signal indicative of a time-varying reflected light intensity.

**12.** The intravascular blood flow sensor system of claim 6, wherein the vibration sensor comprises

- one or more pressure sensors (406) arranged on a surface of the catheter or guidewire, the pressure sensor being arranged and configured to measure a time-varying pressure exerted in the direction perpendicular to the main direction of intravascular blood flow by the vortex-generated blood flow oscillations and to generate and provide the vibration sensor signal in the form of a time-varying electrical signal depending on the measured pressure.

**13.** The intravascular blood flow sensor system of claim 6, wherein

- the bluff part comprises a barrier section (405) that protrudes from the catheter or guidewire (402)in the direction perpendicular to the main direction of intravascular blood flow for generation of vortices propagating along the main direction of intravascular blood flow.

**14.** The intravascular blood flow sensor system of claim 6, further comprising

- a signal communication unit (510) configured to receive the vibration sensor signals and to transmit the vibration sensor signals via a wireless carrier signal to the signal processing unit (510); wherein
- the signal processing unit is further configured to extract the vibration sensor signals from the carrier signal.

**15.** A method (700) for operating a signal processing unit for determining a value of a blood flow quantity characterizing blood flow inside a blood vessel, the method comprising:

- receiving (702) a vibration sensor signal from an intravascular blood flow sensor, the vibration sensor signal comprising a vibration sensor signal component caused by vortex-generated blood flow oscillations of intravascular blood flow;
- determining (704), using the vibration sensor signal, the vibration sensor signal component;
- determining (706), using the vibration sensor signal component, an oscillation frequency of the vortex-generated blood flow oscillations; and
- determining, using the oscillation frequency of the vortex-generated blood flow oscillations, and providing (708) the value of the blood flow quantity.

FIG. 1A

FIG. 1B

FIG. 2

FIG. 3

EP 3 378 381 A1

FIG. 4

FIG. 5

EP 3 378 381 A1

FIG. 6

700

702

704

706

708

FIG. 7

Europäisches
Patentamt

European
Patent Office

Office européen
des brevets

# EUROPEAN SEARCH REPORT

Application Number

EP 17 16 2723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/274712 A1 (SCHECTER STUART O [US]) 17 October 2013 (2013-10-17)<br>* paragraph [0066] *<br>* paragraph [0065] *<br>* paragraph [0070]; figure 2 *<br>* paragraph [0071]; figure 3 *<br>* paragraph [0074] - paragraph [0075]; figure 4 *<br>* paragraph [0094] *<br>* paragraph [0096] *<br>* paragraph [0099] *<br>* paragraph [0116] *<br>----- | 1-15 | INV.<br>A61B5/00<br>A61B5/026<br>A61B5/145<br><br>ADD.<br>A61B18/00 |
| X | US 2014/276124 A1 (CHOLETTE MARTIN [US] ET AL) 18 September 2014 (2014-09-18)<br>* paragraph [0035] - paragraph [0037]; figure 1 *<br>* paragraph [0041] *<br>* paragraph [0052] - paragraph [0053]; figure 2 *<br>* paragraph [0064] - paragraph [0065] *<br>* paragraph [0066] *<br>* paragraph [0069] *<br>----- | 1-9,14, 15 | |
| T | Jason Foust: "Blood flow simulation past a catheter positioned in the SVC-IVC-RA junction : steady and unsteady flow considerations",<br>,<br>30 April 2004 (2004-04-30), XP055403678,<br>Retrieved from the Internet:<br>URL:http://preserve.lehigh.edu/cgi/viewcontent.cgi?article=1846&context=etd<br>[retrieved on 2017-09-04]<br>* page 36 - page 37 *<br>----- | 1-15 | TECHNICAL FIELDS SEARCHED (IPC)<br><br>A61B |

The present search report has been drawn up for all claims

| Place of search | Date of completion of the search | Examiner |
|---|---|---|
| The Hague | 19 September 2017 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 16 2723

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2017

| Patent document cited in search report | Publication date | Patent family member(s) | | Publication date |
|---|---|---|---|---|
| US 2013274712 A1 | 17-10-2013 | NONE | | |
| US 2014276124 A1 | 18-09-2014 | US | 2014276124 A1 | 18-09-2014 |
| | | WO | 2014149553 A1 | 25-09-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140276137 A1